(19) Europäisches Patentamt
European Patent Office
Office européen des brevets

(11) **EP 3 965 793 B1**

(12) **EUROPEAN PATENT SPECIFICATION**

(45) Date of publication and mention
of the grant of the patent:
**01.05.2024 Bulletin 2024/18**

(21) Application number: **20725487.1**

(22) Date of filing: **08.05.2020**

(51) International Patent Classification (IPC):
*A61K 38/08* (2019.01)      *A61P 31/20* (2006.01)
*A61P 31/22* (2006.01)      *A61K 38/17* (2006.01)

(52) Cooperative Patent Classification (CPC):
**A61K 38/08; A61K 38/1729; A61P 31/20;
A61P 31/22**

(86) International application number:
**PCT/EP2020/062882**

(87) International publication number:
**WO 2020/229349 (19.11.2020 Gazette 2020/47)**

(54) **PEPTIDES FOR USE IN THERAPY OR PROPHYLAXIS OF HERPESVIRIDAE-INFECTIONS**

PEPTIDE ZUR VERWENDUNG IN DER THERAPIE ODER PROPHYLAXE VON
HERPESVIRIDAE-INFEKTIONEN

PEPTIDES DESTINÉS À ÊTRE UTILISÉS DANS LA THÉRAPIE OU LA PROPHYLAXIE
D'INFECTIONS PAR LE VIRUS DE L'HERPÈS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.05.2019 DE 102019112244**

(43) Date of publication of application:
**16.03.2022 Bulletin 2022/11**

(73) Proprietor: **Novozymes A/S
2880 Bagsværd (DK)**

(72) Inventors:
• **WEHKAMP, Jan
Hopewell, NJ 08525 (US)**
• **SCHINDLER, Michael
72770 Reutlingen (DE)**
• **BOEFFERT, Rebecca
72070 Tuebingen (DE)**
• **BUSINGER, Ramona
72072 Tuebingen (DE)**
• **EHMANN, Dirk
72074 Tuebingen (DE)**

(74) Representative: **Høiberg P/S
Adelgade 12
1304 Copenhagen K (DK)**

(56) References cited:
• **A. WANG ET AL: "Enhancement of Antiviral
Activity of Human Alpha-Defensin 5 against
Herpes Simplex Virus 2 by Arginine Mutagenesis
at Adaptive Evolution Sites", JOURNAL OF
VIROLOGY, vol. 87, no. 5, 1 March 2013
(2013-03-01), pages 2835-2845, XP055713227, US
ISSN: 0022-538X, DOI: 10.1128/JVI.02209-12**
• **EHSAN HAZRATI ET AL: "Human [alpha]- and
[beta]-Defensins Block Multiple Steps in Herpes
Simplex Virus Infection", THE JOURNAL OF
IMMUNOLOGY, vol. 177, no. 12, 15 December
2006 (2006-12-15), pages 8658-8666,
XP055713282, US ISSN: 0022-1767, DOI:
10.4049/jimmunol.177.12.8658**
• **DATABASE CA [Online] CHEMICAL ABSTRACTS
SERVICE, COLUMBUS, OHIO, US; 9 July 2020
(2020-07-09), Third Military Medical University,
PLA, Peop. Rep. China: "Human .alpha.-defensin
5 mutant polypeptide with antiviral activity and
its preparation and application", XP002799684,
retrieved from STN Database accession no.
2010:610199 & WO 2011/060613 A1 (UNIV PLA
3RD MILITARY MEDICAL [CN]; WANG JUNPING
[CN] ET AL.) 26 May 2011 (2011-05-26)**

- **MEE SOOK PARK ET AL: "Towards the Application of Human Defensins as Antivirals", BIOMOLECULES & THERAPEUTICS, vol. 26, no. 3, 1 May 2018 (2018-05-01), pages 242-254, XP055713294, ISSN: 2005-4483, DOI: 10.4062/biomolther.2017.172**
- **BÖFFERT REBECCA ET AL: "The human [alpha]-defensin-derived peptide HD5(1-9) inhibits cellular attachment and entry of human cytomegalovirus", ANTIVIRAL RESEARCH, ELSEVIER BV, NL, vol. 177, 21 March 2020 (2020-03-21), XP086133704, ISSN: 0166-3542, DOI: 10.1016/J.ANTIVIRAL.2020.104779 [retrieved on 2020-03-21]**

Remarks:

The complete document including Reference Table(s) and the Sequence Listing(s) can be downloaded from the EPO website

**Description**

The present invention relates to specific peptides and peptide sequences and their use in medical therapy and/or prophylaxis or viral *Herpesviridae* infections in mammals.

*Herpesviridae* are a diverse family of DNA viruses that cause infections and diseases in animals, including humans, and comprise species such as Human Cytomegalovirus (HCMV; HHV-5), Herpes Simplex Virus 1 (HSV-1; HHV-1), Herpes Simplex Virus 2 (HSV-2; HHV-2), Varicella Zoster Virus (VZV; HHV-3), Epstein-Barr Virus (EBV; HHV-4), Human Herpes Virus 6 (HHV-6), Human Herpes Virus 7 (HHV-7), or Kaposi's Sarcoma-Associated Herpesvirus (KSHV; HHV-8). Among these, HSV-1 and HSV-2 (both of which can cause orolabial herpes and genital herpes), varicella zoster virus (the cause of chickenpox and shingles), Epstein-Barr virus (implicated in several diseases, including mononucleosis and some cancers), and cytomegalovirus - are extremely widespread among humans. More than 90% of adults have been infected with at least one of these, and a latent form of the virus remains in almost all humans.

The Herpesviridae represent enveloped viruses that infect hosts for life by establishing latency until virus reactivation due to immunosuppression, stress or other cues.

HCMV is a ubiquitous opportunistic pathogen that belongs to B*etaherpesviridae.* The virulence of this pathogen is directly linked to the immune status of its host. Primary HCMV infection is generally asymptomatic in immunocompetent individuals. After primary HCMV infection, the virus establishes lifelong latency within the host and periodically reactivates with little pathological consequences. In contrast, HCMV infection in immunocompromised patients, such as AIDS patients, and solid organ and allogeneic stem cell transplantation recipients causes serious disease.

HCMV is also the leading cause of congenital viral infection most often resulting from a primary infection of the mother during or right before pregnancy that leads to spontaneous abortion, premature delivery, intrauterine growth restriction (IUGR), or pre-eclampsia. The risk of primary infection in a seronegative mother is 1 to 4%, which carries a 30 to 40% risk of congenital infection. The majority of congenitally infected babies are asymptomatic at birth; however, 10 to 17% will subsequently develop hearing defects or neurodevelopmental sequelae.

HCMV infection is highly prevalent in the population due to the ability of the virus to efficiently transmit between hosts that harbor and periodically shed the virus. HCMV is transmitted through the direct exposure to infected body secretions, including saliva, urine and milk. Following infection, HCMV enters the bloodstream and spreads to various organs including kidney, liver, spleen, heart, brain, retina, and others.

The currently primarily used therapeutic strategy for treating HCMV infections are therapies inhibiting the viral replication within the cells. As such, the primary existing therapeutic agent for treating a HCMV infection represents the medicament Ganciclovir, a nucleoside-analogue, which, after phosphorylation via viral kinases, acts as a DNA-polymerase inhibitor. In case of a Ganciclovir-resistance mediated through a viral mutation in the UL97 gene, alternatives such as Foscarnet and Cidofovir are used as virostatic agent, which - as DNA-polymerase inhibitors - also inhibit virus replication. Also currently used in the treatment of HCMV-infections is the viral terminase inhibitor Letermovir, which, however, has no significant activity against other herpesvirus or nonhuman CMV.

The mentioned therapeutic agents all share the drawback that their appliance is involved with major side effects, such as myelo- and nephro-toxicity.

Currently, in particular for pregnant women and newborns there is no evidence-based therapeutic approach.

Thus, it is an object of the present invention to provide for a new therapeutic agent useful and effective for the treatment/inhibition or prevention of *Herpesviridae* infections, in particular of *Betaherpesviridae* infections, and more particularly in of HCMV infections in mammals, in particular humans.

According to the invention, this and other problems are solved by the provision of a peptide for use in treating, inhibiting and/or preventing viral infections in mammals, wherein the viral infection is caused by Cytomegalovirus wherein the peptide comprises a sequence having the following formula I:

$$A1\text{-}B1\text{-}X1\text{-}C1\text{-}X2\text{-}Z1\text{-}B2\text{-}A2\text{-}Z2 \qquad \text{(Formula I)}$$

wherein

A1 and A2 are identical or different, and each A1 and B2 is a nonpolar amino acid having an aliphatic or basic side chain, and each is preferably alanine or glycine, B1 and B2 are identical or different, and each B1 and B2 is a polar amino acid and having a hydroxylated side chain, and is preferably selected from serine, threonine, or tyrosine, C1 is an polar amino acid having a aromatic side chain, and is preferably tyrosine, X1 and X2 each is cysteine, and Z1 and Z2 each is arginine.

With the present invention, viral Herpesviridae-infections, in particular those with HCMV, can be efficiently treated and/or prevented, since it has been demonstrated that the peptide(s) provided herein have an antiviral effect, displaying no toxicity on different human cells, even at high concentrations. Also, with the peptide(s) according to the invention, an infection with clinical and multi-resistant HCMV-isolates was inhibited to the same extent as an infection with a laboratory-adapted strain.

The peptide(s) as provided herein have a sequence that is derived from a defensin, i.e. human defensin (HD) 5. Defensins are endogenously produced antimicrobial peptides, which belong to the innate immune system. They are small cationic molecules, characterized by three conserved disulphide bonds and represent a main group of AMPs. To date, six alpha-defensins have been identified in humans, namely the four Human Neutrophil Peptides (HNP) 1, 2, 3 and 4, and the two Human Defensin (HD) 5 and 6. While the HNPs form part of the armoury of neutrophils, where they participate in systemic innate immunity, the HDs are expressed in intestinal Paneth cells. In the small intestine, Paneth cells play a key role in balancing the microbiota composition and in protecting the host from invading pathogens by secretion of a variety of AMPs but most abundantly the two $\alpha$-defensin 5 (HD 5) and -6 (HD 6).

The anti-microbial activity of alpha-defensins has been intensively studied in the past, and it has been acknowledged that alterations in their specific sequences can contribute to major changes of their activity, and may even lead to a complete loss of antimicrobial activity. Also, it is known that some of the defensins are proteolytically cleaved, with some of the fragments still retaining antimicrobial activity.

Within the present invention, peptide sequences of HD-5 have been identified which, in antiviral tests, showed to have an increased antiviral effect on HCMV as compared to the full length peptide, and certain other peptide fragments.

Also, the peptide according to the invention offers the advantage that in contrast to the therapeutic agents currently employed primarily, the peptide according to the invention already inhibits the virus of entering the cell, while medicaments such as Ganciclovir merely inhibit viral replication within an already infected cell.

Accordingly, within the present invention, it has been shown that for an antiviral effect, the amino acids $X_1$ and $X_2$ as designated in the above formula (I) need to be cysteine residues, and the amino acids $Z_1$ and $Z_2$ as designated in the above formula (I) need to be arginine.

Further, according to a preferred embodiment, the peptide for use has an amino acid sequence that comprises 9 successive amino acids having the sequence ATCYCRTGR (SEQ ID No. 1) or the reverse sequence of SEQ ID No. 1, RGTRCYCTA (SEQ ID No. 2) of the attached sequence listing. In some embodiments, the variant differs from the corresponding portion of SEQ ID NO: 1 only by one or more conservative substitutions, wherein the amino acids $X_1$ and $X_2$ as designated in the above formula (I) are not substituted and are cysteine residues, and the amino acids $Z_1$ and $Z_2$ as designated in the above formula (I) are also not substituted and are arginine.

The peptides of the present invention can include other amino acid sequences flanking the SEQ ID No. 1 sequence (e.g., at the amino and/or carboxyl terminus thereof). Such additional sequences, as well as substitutions in the segment of SEQ ID NO: 1, can be used to modify or optimize the physical, physiological, or chemical characteristics of the peptide for use in a pharmaceutical composition for inhibiting *Herpesviridae* infections. For example, the additional sequences or substitutions can stabilize the peptide under physiological conditions, or can be used to enhance compatibility of the peptide with a preferred delivery vehicle or carrier.

As used herein, the term "conservative substitution" refers to the presence of an amino acid residue in the sequence of the peptide that is different from, but is in the same class of amino acid as the "wild-type" residue of SEQ ID No. 1.

In this regard, conservative substitutions include a nonpolar (hydrophobic) amino acid residue replacing a nonpolar amino acid residue, an aromatic amino acid residue replacing an aromatic amino acid residue, or a polar (hydrophilic) amino acid residue replacing a polar amino acid residue (e.g., a polar uncharged residue replacing a polar uncharged residue, a charged residue replacing a charged residue, a polar uncharged residue (e.g., Asn or Gln) replacing a charged residue (e.g., Asp or Glu), or a charged residue replacing a polar uncharged residue).

As used herein, the term "nonpolar amino acid residue" (also sometimes referred to as a hydrophobic residue) refers to alanine, valine, leucine, isoleucine, proline, and aromatic residues; the term "aromatic amino acid residue" refers to phenylalanine, tyrosine, and tryptophan; the term "polar amino acid residue" (also sometimes referred to as a hydrophilic residue) refers to polar uncharged residues such as serine, threonine, cysteine, methionine, asparagine and glutamine as well as charged residues, such as the negatively charged (acidic) amino acid residues (aspartic acid and glutamic acid), and the positively charged (basic) amino acid residues (lysine, arginine, and histidine). Glycine is can be included as either a polar or a nonpolar amino acid residue with respect to conservative substitutions.

According to another preferred embodiment, the peptide for use consists of the SEQ ID No. 1 or the reverse sequence of SEQ ID No. 1, i.e. RGTRCYCTA (SEQ ID No. 2) of the attached sequence listing.

Still according to another aspect, the peptide of the invention is a chemically or enzymatically synthesized peptide or a recombinant peptide.

A wide variety of methods for chemically synthesizing peptides are known in the art; while the chemical synthesis of peptides can be carried out using classical solution-phase techniques, these have been replaced in most research and development settings by solid-phase methods. An overview of peptide synthesis can be found, e.g. in Stawikowski et al., ("Introduction to peptide synthesis", Cur. Prot. Prot. Sci., (2012), suppl. 69, 18.1.1-18.1.13).

The peptide of the invention can also be generated by recombinantly expressing the peptide. Recombinant peptides can be produced using bacterial (in particular *E. coli* or yeast), mammalian, or insect cell expression systems. A recombinant peptide is produced through recombinant DNA technology involving the insertion of the DNA encoding the peptide of the invention into bacterial, mammalian or insect cells, expressing the peptide in these cells. The expressed peptide can be purified from these cells, and, thus is an "isolated" peptide. An overview over recombinant microbial synthesis can be found, e.g., in Wegmuller and Schmid, "Recombinant Peptide Production in Microbial Cells", Current Organic Chemistry, 18(8) (2014).

A "recombinantly expressed" or "biologically expressed" peptide, within the present invention, shall encompass the expression of the peptide(s) for use according to the invention by a genetically engineered host cell that has been modified to express said peptide(s). As used herein, the term "host cell" is presently defined as a cell which has been transformed or transfected, or is capable of transformation or transfection by an exogenous polynucleotide sequence encoding the peptide for use according to the invention. A variety of host-expression vector systems may be utilized to express the gene coding a peptide for use according to the invention. Such host-expression systems represent vehicles by which the coding sequences of interest may be produced and subsequently purified, but also represent cells which, when transformed or transfected with the appropriate nucleotide coding sequences, exhibit the peptide for use gene product of the invention *in situ.*

For recombinant production, host cells can be genetically engineered to incorporate expression systems or portions thereof or polynucleotides encoding the peptides for use of the invention. Introduction of a polynucleotide into the host cell can be effected by methods described in many standard laboratory manuals, such as Davis et al., Basic Methods in Molecular Biology, (1986), and Sambrook *et al.,* 1989.

Thus, the polynucleotide encoding the peptide according to the invention, may, e.g., be comprised in a vector which is to be stably transformed/transfected into host cells. In the vector, the polynucleotide encoding the peptide(s) of the invention is under control of an, e.g., inducible promoter, so that the expression of the gene/polynucleotide can be specifically targeted, and, if desired, the gene may be overexpressed in that way.

A great variety of expression systems can be used to produce the polypeptides of the invention. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cosmids and phagemids. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook *et al.,* see above.

According to an aspect of the invention, the peptide of the invention (or the peptidomimetic or the pharmaceutical composition of the invention, as explained below) is used for treating, preventing and/or inhibiting a viral infection caused by a member of the *Betaherpesviridae.*

The *Betaherpesviridae* are a subfamily of the *Herpesviridae,* with mammals serving as natural hosts for these viruses. There are currently 18 species in this subfamily, divided among 4 genera. Diseases associated with this subfamily include: human cytomegalovirus (HCMV), also known as Human Herpes Virus 5 (HHV-5), causing congenital CMV infection; Human Herpes Virus 6A and 6B (HHV-6A and HHV-6B), causing *Roseola Infantum* or *Exanthema Subitum*); and Human Herpes Virus 7 (HHV-7), causing symptoms analogous to those of HHV-6A and -6B.

While the Human Cytomegalovirus (HCMV, HHV-5) has a large impact on immune parameters in later life and likely contributes to increased morbidity and eventual mortality, Human Herpes Virus 6A (HHV-6A) has been described as more neurovirulent, and, as such is more frequently found in patients with neuroinflammatory diseases such as multiple sclerosis. Both human herpesvirus 6B (HHV-6B) and human herpesvirus 7 (HHV-7), as well as other viruses, can cause a skin condition in infants known as *Roseola Infantum* or *Exanthema Subitum* (rose rash of infants) or the sixth diseas.

According to another aspect of the invention, the peptide of the invention is used for treating, preventing and/or inhibiting a viral infection caused by at least one of the following: human Cytomegalovirus (HCMV; HHV-5), Herpes Simplex Virus 1 (HSV-1; HHV-1), Herpes Simplex Virus 2 (HSV-2; HHV-2), Varicella Zoster Virus (VZV; HHV-3), Epstein-Barr Virus (EBV; HHV- 4), Human Herpes Virus 6 (HHV-6), including Human Herpes Virus 6A and 6B), Human Herpes Virus 7 (HHV-7), or Kaposi's sarcoma-Associated Herpesvirus (KSHV; HHV-8).

According to a preferred embodiment, the peptide according to the invention is used in treating, preventing and/or inhibiting a viral infection caused by the human Cytomegalovirus (HCMV; HHV-5).

According to yet another preferred embodiment, the peptide of the invention is used in treating, preventing and/or inhibiting a viral infection caused by a multiresistant HCMV strain.

According to another aspect, the peptide of the invention or a peptidomimetic of the invention is used in combination with one or more other therapeutic agent, e.g., as a combination therapy.

For example, a pharmaceutical composition can include one or more other anti-infective agents in addition to the peptide or peptidomimetic of the invention, such as, for example, an antiviral protease enzyme inhibitor (PI), a virus DNA or reverse transcriptase (RT) polymerase inhibitor, another virus/cell fusion inhibitor, a virus integrase enzyme inhibitor, a virus/cell binding inhibitor, a herpes virus DNA polymerase inhibitor, such as acyclovir, ganciclovir, cidofovir, and the like), a herpes virus protease inhibitor, a herpes virus fusion inhibitor, a herpes virus binding inhibitor, a ribonucleotide reductase inhibitor, and the like. The additional therapeutic agent will be included in the compositions within a therapeutically useful and effective concentration range, as determined by routine methods that are well known in the medical and pharmaceutical arts. The concentration of any particular additional therapeutic agent may be in the same range as is typical for use of that agent as a monotherapy, or the concentration may be lower than a typical monotherapy concentration if there is a synergy when combined with a peptide of the present invention.

The peptide for use as claimed in any of claims 1 to 8, wherein the peptide is used in a concentration of at least about 1 nM, 10 nM, 100 nM, 1 μM, 10 μM, 20 μM, 30 μM, 40 μM, 50 μM, 60 μM, 70 μM, 80 μM, 90 μM, 100 μM, 110 μM, 120 μM, 130 μM, 140 μM, or about 150 μM.

According to one aspect of the invention, the peptide is used in a concentration of between about 1 μM and about 150 μM, preferably of about 10 μM to 150 μM, 20 μM to about 150 μM, 30 μM to about 150 μM, 40 μM to about 150 μM, or 50 μM to about 150 μM.

According to another aspect, the invention also concerns a peptidomimetic for use in treating, inhibiting and/or preventing viral infections in mammals, wherein the viral infection is caused by a member of the family *Herpesviridae,* in particular by a member of the family *Betaherpesviridae,* and wherein the peptidomimetic is derived from the peptide for use of the invention, and is preferably derived from the peptide having the SEQ ID No. 1.

A peptidomimetic is a small protein-like chain designed to mimic a peptide, i.e. the peptide according to the invention. Peptidomimetics typically arise either from modification of an existing peptide, i.e. from the peptide according to the invention, or by designing similar systems that mimic peptides, such as peptoids and β-peptides (= "derived from the peptide having SEQ ID No. 1). Irrespective of the approach, the altered chemical structure is designed to advantageously adjust the molecular properties such as, stability or biological activity. The modifications involve changes to the peptide that will not occur naturally (such as altered backbones and the incorporation of nonnatural amino acids).

The invention also concerns a pharmaceutical composition for use in treating, preventing and/or inhibiting a *Herpesviridae* infection, in particular a *Betaherpesviridae* infection, wherein the pharmaceutical composition comprises a peptide and/or the peptidomimetic of the invention, and a pharmaceutically acceptable carrier, vehicle or diluent.

The pharmaceutical composition can also include excipients, preservatives, and other pharmaceutically useful materials, e.g., to provide storage stability, or to impart desirable physical properties to the composition. The pharmaceutical compositions of the present invention can be formulated with other therapeutic agents, such as antiviral agents, if desired.

Presently, and as generally understood in the field, a "pharmaceutically acceptable carrier" is understood to mean any excipient, additive, or vehicle that is typically used in the field of the treatment of the mentioned diseases and which simplifies or enables the administration of the product according to the invention to a living being, and/or improves its stability and/or activity. The pharmaceutical composition can also incorporate binding agents, diluting agents or lubricants. The selection of a pharmaceutical carrier or other additives can be made on the basis of the intended administration route and standard pharmaceutical practice. As pharmaceutical acceptable carrier use can be made of solvents, extenders, or other liquid binding media such as dispersing or suspending agents, surfactant, isotonic agents, spreaders or emulsifiers, preservatives, encapsulating agents, solid binding media, depending upon what is best suited for the respective dose regime and is likewise compatible with the compound according to the invention. An overview of such additional ingredients can be found in, for example, Rowe (Ed.) et al.: Handbook of Pharmaceutical Excipients, 7th edition, 2012, Pharmaceutical Press.

The pharmaceutical composition can, e.g., comprise an aqueous buffer at a physiologically acceptable pH (e.g., pH 7 to 8.5), a polymer-based nanoparticle vehicle, a liposome, and the like. The pharmaceutical compositions can be delivered in any suitable dosage form, such as a liquid, gel, solid, cream, or paste dosage form. In one embodiment, the compositions can be adapted to give sustained release of the peptide.

In some embodiments, the pharmaceutical compositions can include forms suitable for oral, rectal, nasal, topical, (including buccal and sublingual), transdermal, vaginal, or parenteral (including intramuscular, subcutaneous, and intravenous) administration, in a form suitable for administration by inhalation or insufflation, or injection into amniotic fluid. The compositions can, where appropriate, be conveniently provided in discrete dosage units. The pharmaceutical compositions of the invention can be prepared by any of the methods well known in the pharmaceutical arts. Some preferred modes of administration include intravenous (iv), topical, subcutaneous, and injection into amniotic fluid.

According to another aspect, the invention also relates to a method of treating, preventing, or inhibiting a *Herpesviridae* infection, in particular a *Betaherpesviri*dae infection, comprising administering a therapeutically effective amount of the peptide of the invention, the peptidomimetic of the invention or the pharmaceutical composition of the invention to a subject.

While not being limited thereto, the method can be particularly effective for treating immuno-compromised subjects, pregnant women, newborns, e.g., for HCMV infections. The peptides will be administered to a subject at a therapeutically useful and effective dosage range, as determined by routine methods that are well known in the medical and pharmaceutical arts.

As mentioned for the concentration for the peptide above, for example, a typical unit dosage will deliver an amount of the peptide in the range of at least about 1 milligram to about 1000 mg, preferably at least about 10 milligrams to about 100 milligrams. The peptide can be administered in a single unit dose, or in multiple unit doses delivered, for example on a per hour, per day, per week, or per month, schedule for a fixed or indefinite period of time. The particular dosage and administration protocol will vary depending on the dosage form, the particular peptide, the type of infection, the severity of the infection, the physical condition of the subject, and other factors that are well known in the medical and pharmaceutical arts.

According to the invention, the peptide, the peptidomimetic, the pharmaceutical composition, and/ or in the method of the invention, is administered to a subject that it selected from an immuno-compromised subject, pregnant women, newborns or infants, or organ-transplant-recipients.

As mentioned at the outset, Herpesviridae infections, in particular Be*taherpesviridae* infections, in particular HCMV infections, are particularly critical for pregnant women and their unborn children. HCMV infections are a frequent cause for birth malformations such as hearing loss or neurodevelopmental disorders. With the peptide, peptidomimetic, pharmaceutical compositions and method according to the invention, the risks of such infections and their consequences can be avoided.

According to the invention, the peptidomimetic, the pharmaceutical composition, or the method of the invention, as already the peptide of the invention, is used for treating, preventing or inhibiting an infection caused be at least one of the following: Cytomegalovirus (HCMV; HHV-5), Herpes Simplex Virus 1 (HSV-1; HHV-1), Herpes Simplex Virus 2 (HSV-2; HHV-2), Varicella Zoster Virus (VZV; HHV-3), Epstein-Barr Virus (EBV; HHV- 4), Human Herpes Virus 6 (HHV-6), Human Herpes Virus 7 (HHV-7), or Kaposi' sarcoma-Associated Herpesvirus (KSHV; HHV-8).

The peptides, pharmaceutical compositions, peptidomimetics and methods of the present invention can beneficially be used to treat any subject/patient suffering from or at risk of developing a *Herpesviridae* infection, in particular a *Betaherpesviridae* infection, in particular a HCMV infection, or outbreak thereof. The peptides, pharmaceutical compositions, peptidomimetics and methods of this invention are particularly useful for immuno-compromised patients (retinitis patients, transplant patients, allogeneic stem cell transplantation recipients), as well as to reduce viral load in pregnant women, thereby reducing spread of a herpesvirus (in particular HCMV and the like) to the placenta and subsequently to the fetus. Additionally, these peptides, pharmaceutical compositions, peptidomimetics and methods also can be used for treating CMV-infected neonates to reduce the risk of development of sensorineural hearing loss.

According to a preferred embodiment of the invention, the peptides, pharmaceutical compositions, and/or peptidomimetics of the invention can be administered orally, rectally, nasally, topically, (including buccally and sublingually), transdermally, vaginally, or parenterally (including intramuscularly, subcutaneously, and intravenously).

The dosage level for administering the peptide or peptidomimetic to a subject/patient will be an amount sufficient to provide a therapeutically useful outcome (e.g., suppression of a *Herpesviridae-member* outbreak, prevention of an active infection after known exposure to a member of the *Herpesviridae,* and the like). The determination of a therapeutically effective amount of a peptide or peptidomimetic of the invention is also within the level of ordinary skill of medical and pharmaceutical professionals.

The following Examples are provided to illustrate certain aspects and features of the isolated peptides, pharmaceutical compositions, and methods of the present invention.

It is to be understood that the before-mentioned features and those to be mentioned in the following cannot only be used in the combination indicated in the respective case, but also in other combinations or in an isolated manner without departing from the scope of the invention.

The invention is now further explained by means of embodiments resulting in additional features, characteristics and advantages of the invention. The embodiments are of pure illustrative nature and do not limit the scope or range of the invention.

The features mentioned in the specific embodiments are also features of the invention in general, which are not only applicable in the respective embodiment but also in an isolated manner in the context of any embodiment of the invention.

The invention is also described and explained in further detail by referring to the following drawings:

Further advantages will be shown in the following figures and description of the Examples, which represent exemplary embodiments only, and shall not be construed as limiting the invention.

In the figures:

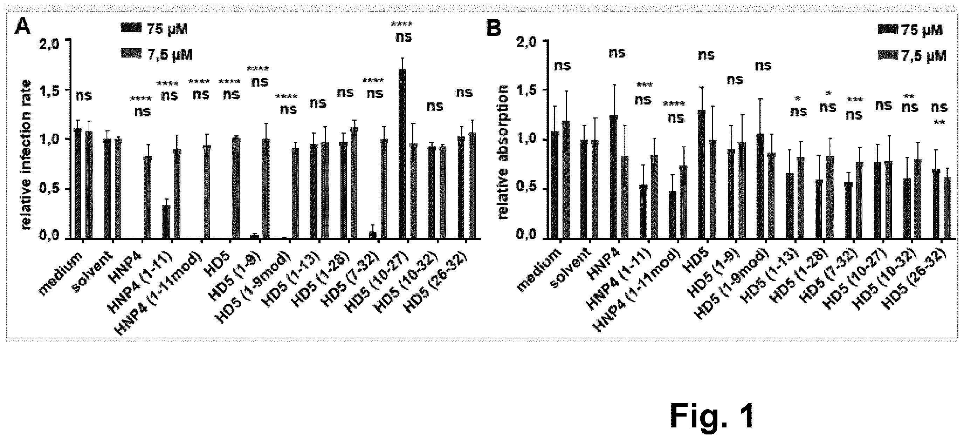

**Fig. 1 (A)** shows a diagram displaying the results of experiments treating HCMV-infected HFF with different concentrations of HD 5-derived peptides; HFF were infected with TB40E-ΔUL16-EGFP and treated with peptides in different concentrations. After 40 h of incubation, cells were fixed and stained with DAPI, and the infection rate was analysed (GFP+/DAPI+, n=1); (B) shows a diagram displaying results of the MTT assay; HFF were treated with peptides in different concentrations; after 40 h of incubation, MTT stock solution was added and crystals were dissolved; absorption was measured (n=3);

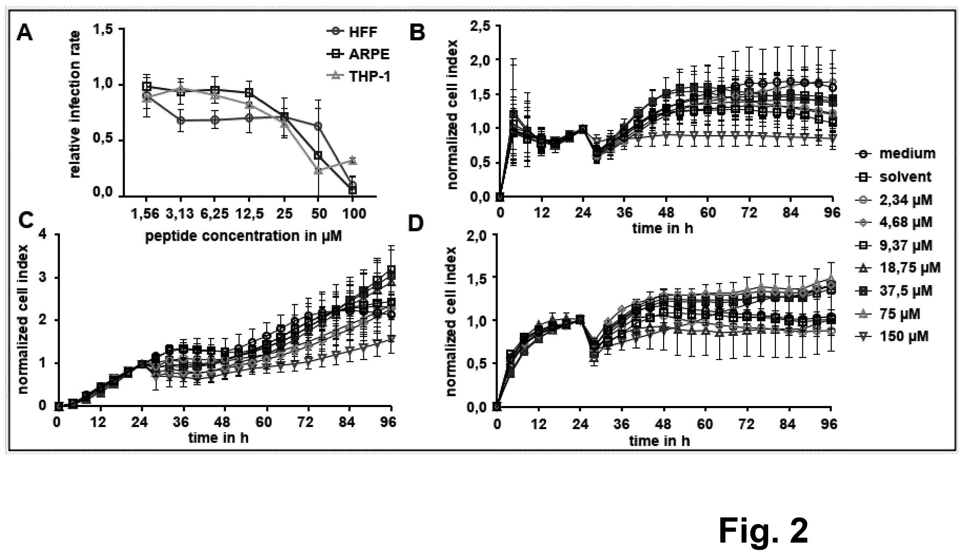

**Fig. 2 (A)** shows a diagram displaying the results of experiments where different cell types were infected with TB40E-ΔUL16-EGFP and treated with HD5 (1-9) in different concentrations. After 40 h of incubation cells were fixed and an antibody staining for IE-antigens was performed; afterwards, nuclei were stained with DAPI. The infection rate was analysed (IE+/DAPI+, n=3, THP-1 n=1); **(B - D)** shows a diagram displaying the results of experiments, were different cell types were treated with the different peptides testing their toxicity: HFF **(B)**, ARPE **(C)** and macrophages **(D)** were seeded in a culture dish with a microelectrode network on the bottom; after 24 h cells were treated with peptides in different concentrations; impedance was measured via xCELLigence every 30 min for 72h (n=3, macrophages n=1);

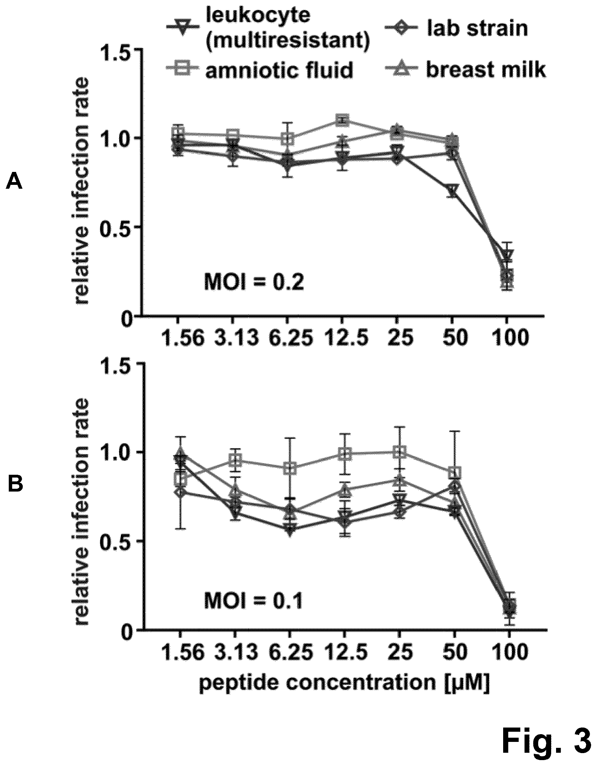

**Fig. 3** shows a diagram displaying results of experiments testing HD5 (1-9) on different clinical HCMV-isolates; HFF were infected with different clinical HCMV isolates and TB40/E-ΔUL16-eGFP as reference strain with an MOI of 0.2 **(A)** or 0.1 **(B)** and treated with HD5 (1-9) (SEQ ID No. 1) in different concentrations. After 40 h incubation, cells were fixed and HCMV-infected cells identified by IE1/2 antigen staining. Nuclear staining was done with DAPI. Infection rates were measured by imaging with a microplate imager and automated counting of DAPI+ and IE1/2+ cells. The graphs show the calculated infection rate (IE1/2+/DAPI+, normalized to medium only, mean $\pm$ SD from duplicate infections each).

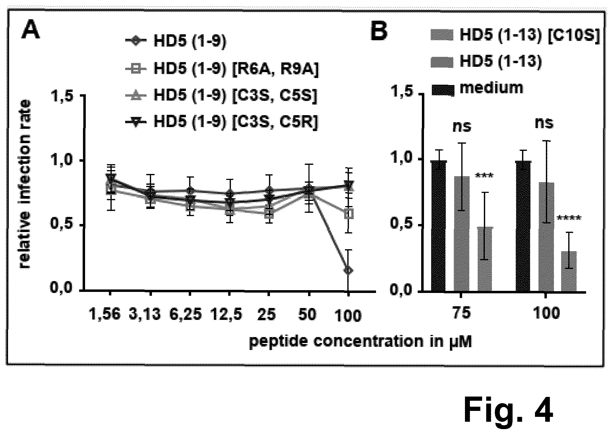

**Fig. 4 (A)** shows a diagram displaying the results of experiments where HFF were infected with TB40E-ΔUL16-EGFP and treated with HD5 (1-9) and its derivatives; **(B)** HFF were infected with TB40E-ΔUL16-EGFP and treated with HD5 (1-9) and its derivatives in different concentrations; after 40h of incubation, cells were fixed and an antibody staining for IE-antigens ware performed; afterwards nuclei were stained with DAPI; the infection rate was analysed (IE+/DAPI+, n=3); HD5 (1-9): ATCYCRTGR (SEQ ID No. 1); HD5 (1-13): ATCYCRTGRCATR (SEQ ID No. 3); ns: not significant, **** $p < 0.0001$, *** $p < 0.001$; and

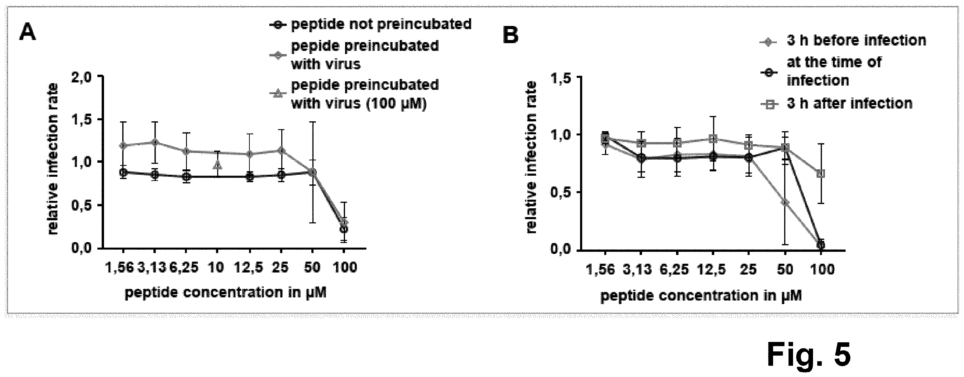

**fig. 5 (A)** shows a diagram displaying the results of experiments where HFF were infected with TB40E-ΔUL16-EGFP and treated with HD5 (1-9) in three different concentrations; in the first condition there was no preincubation of virus and peptide; in the second condition the peptide in the corresponding concentrations was preincubated with the peptide, in the third condition the peptide was preincubated with the virus in 100 μM and then diluted to 10 μM; preincubation was 1 h at 37°C; after 40h of incubation, cells were fixed and an antibody staining for IE-antigens ware performed; afterwards nuclei were stained with DAPI; the infection rate was analysed (IE+/DAPI+, n=3); **(B)** in the first condition, HFF were preincubated with peptide in different concentrations 3 h at 37°C, in the second condition the peptide was added at the time of infection and in the third condition the peptide was added 3 h after infection; HFF were infected with TB40E-ΔUL16-EGFP and after 40 h of incubation cells were fixed and an antibody staining for IE-antigens ware performed; afterwards nuclei were stained with DAPI; the infection rate was analysed (IE+/DAPI+, n=3).

**Examples:**

**Materials and Methods**

**Infection assays:**

For the infection assays, the cells were sown in 96-well plates. Human foreskin fibroblasts (HFF) and ARPE-19 cells (Adult Retinal Pigment Epithelial cell line-19) were seeded with 10 000 cells in 200 μl medium per well, macrophages with 20 000 cells in 200 μl medium per well and THP with 50 000 cells in 200 μl per well. The cells were incubated overnight at 37° C and 5% $CO_2$ and treated the following day. A medium change to P/S-free medium was performed to avoid interaction between the antibiotics used and the peptide sequences. Afterwards the corresponding peptides were added in different concentrations and immediately afterwards the virus, the final volume was 100 μl per well. The virus of the strain TB40/E-ΔUL16-eGFP was used. TB40-ΔUL16-eGFP is a derivative of the strain TB40, in which the majority of the open reading frame UL16 has been replaced by the open reading frame of eGFP, essentially as described in detail for a homologous mutant of strain AD169 (Tischer et al., "Two-step red-mediated recombination for versatile high-efficiency markerless DNA manipulation in Escherichia coli", Biotechniques, (2006), 40(2):191-7). TB40-delUL16-eGFP expresses eGFP under the control of the early UL16 promotor and has previously been used for FACS analyses (Sinzger et al., "Macrophage cultures are susceptible to lytic productive infection by endothelial-cell-propagated human cytomegalovirus strains and present viral IE1 protein to CD4+ T cells despite late downregulation of MHC class II molecules", J. Gen. Virol., (2006), 87:1853-62). Depending on the experiment, different MOI ("multiplicity of infection") were used, some of which were titrated beforehand. For HFF and ARPE-19, an absolute infection rate of 80 % was targeted. For myeloid cells such a high infection rate could not be achieved, therefore an absolute infection rate of 40 % was aimed at. The required volume of the virus stock was calculated using the titer according to the following formula:

$$\text{Volume in ml} = (1/(\text{titer in IU/ml})) \times (\text{cell number in well} \times \text{MOI})$$

After a further 40 h incubation period, the cells were fixed with 150 $\mu$l 2% paraformaldehyde (PFA) in PBS per well (10 min at 37° C, 20 min at room temperature or overnight in the refrigerator) and permeabilized with 200 $\mu$l -20° C cold 90% methanol in water for 20 min in the refrigerator. Then an antibody staining on IE-positive cells was performed, as this allows a very precise quantification of the infection rate. This was done with HCMV IE E13 as first antibody, of which 100 $\mu$l per well of a 1:1000 dilution in PBS were incubated for 90 min with the cells. As second antibody Goat anti-Mouse IgG (H+L) Alexa Fluor 594 was used, of which per well 100 $\mu$l of a 1:2000 dilution in PBS for 60 min were incubated with the cells. Finally, nuclear staining with DAPI was performed by leaving 100 $\mu$l per well of a 1:20 000 dilution of the DAPI stock solution (2 mg DAPI + 1 ml PBS) in PBS for 8 min at room temperature on the cells. Between each step, the cells were washed three times with PBS. In the last step the PBS was left on the cells and the plate was stored at 4 °C in the refrigerator. Images were taken in the Cytation™ reader (BioTek Instruments), for evaluation. The infection rate was calculated by relating the number of IE positive signals to the number of DAPI stained nuclei. To determine the relative infection rate, the corresponding infection rate was related to the mean infection rate of the untreated or solvent-treated HCMV-infected cells. Technical duplicates or triplicates were used in the experiments.

**Infection assays with clinical isolates**

The antiviral activity of HD5 (1-9) (SEQ ID No. 1) in concentrations from 1.56 $\mu$M to 100 $\mu$M against infection with clinical HCMV isolates was investigated on HFF. In addition to the laboratory-adapted strain TB40/E-$\Delta$UL16-eGFP, which served as reference strain, a breast milk-derived strain, an amniotic fluid-isolated strain and a multidrug-resistant viral isolate from leukocytes of a recipient after the third stem cell transplantation were used. The therapy-naive strain from cell free milk whey (H1241-2016) was derived from a mother of a preterm infant 10 weeks postpartum during end of viral reactivation. The amnion fluid derived virus strain (H2497-2011) was isolated following termination of pregnancy based on severe fetal brain damage. The multidrug resistant CMV isolate (H815-2006) is already described (Gohring et al., "Dynamics of coexisting HCMV-UL97 and UL54 drug-resistance associated mutations in patients after haematopoietic cell transplantation", J. Clin. Virol. 57(1):43-49, 2013). This viral isolate showed the canonical UL97 mutation L595S and an UL54 mutation V715M, leading to drug resistance against GCV, IC50 = 31.5 $\mu$M, and CDV, IC50 = 795 $\mu$M. IC50 value against PFA was 1.8 $\mu$M. All viral isolates were primarily HFF-adapted and propagated in vitro with at least 10 passages to get TCID values of cell free viral supernatants of 105 to 106/ml.

**MTT:**

In this test the cell viability of HFF was tested after treatment with the peptides HNP4, HNP4 (1-11), HNP4 (1-11 mod), HD5, HD5 (1-9), HD5 (1-9mod), HD5 (1-13), HD5 (1-28), HD5 (7-32), HD5 (10-27), HD5 (10-32) and HD5 (26-32) in different concentrations. The assay is based on the reduction of the yellow water-soluble dye MTT (3-(4,5-dimethylthiazol-2-yl)-2,5-diphenyltetrazolium bromide) in blue-violet waterinsoluble 2,3,5-Triphenyltetrazolium chloride with the aid of the pyridine-containing reduction equivalents NADH (reduced form of nicotinamide adenine dinucleotide) and NADPH (reduced form of nicotinamide adenine dinucleotide phosphate) (Berridge et al. "Characterization of the cellular reduction of 3-(4,5-dimethylthiazol-2-yl)-2,5-diphenyltetrazolium bromide (MTT): subcellular localization, substrate dependence, and involvement of mitochondrial electron transport in MTT reduction", Arch. Biochem. Biophys. 303(2): 474-82 (1993)). For the screening tests 10 000 HFF in 200 $\mu$l medium per well were sown in 96-well plates. The cells were incubated overnight at 37° C and 5 % $CO_2$ and treated the following day. A medium change to P/S-free medium was performed to avoid interaction between the antibiotics used and the peptide sequences. Afterwards, the peptides were added, such, that peptide concentrations of 7.5 $\mu$M and 75 $\mu$M were obtained analogous to the screening tests for antiviral activity. The final volume was 100 $\mu$l per well. The plate was incubated for 40 h, followed by a media change to 90 $\mu$l P/S and phenol red free medium. Then 10 $\mu$l per well of MTT stock solution (5 mg MTT + 1 ml phenol red free DMEM) were added and carefully resuspended. After a further 3 h incubation period, the medium was removed and the crystals were dissolved in 100 $\mu$l 0.04 M hydrochloric acid each in isopropanol per well 10 min on the shaker. The absorption was then measured in the Cytation™ reader at 570 and 650 nm. For evaluation, the mean absorption value measured in empty wells was subtracted from all measured absorption values. To determine absolute absorption, the absorption values of the reference wavelength 650 nm were subtracted from the values at 570 nm. To determine the relative absorption, the corresponding value was related to the mean value of the absorption of the HFF treated with 0,01 % acetic acid. Technical triplicates were used.

**Impedance measurement:**

The "xCELLigence" system (ACEA Biosciences Inc.) was used for a more detailed toxicity study. The principle is based on a measurement of the electrical resistance caused by adhesive cells on the bottom of a 96-well plate equipped with microelectrodes. Cell proliferation causes an increase in resistance due to the partial isolation of the electrodes, while events leading to altered cell morphology or cell detachment lead to reduced resistance (Diemert et al. "Impedance measurement for real time detection of neuronal cell death", J Neurosci Methods, 203(1):69-77, 2012 )). The experiments were performed with HFF, ARPE-19 and macrophages, the appropriate cell numbers were determined in a preliminary experiment without treatment with peptides.

On the first day 10 000 HFF or ARPE-19 or 20 000 macrophages per well were sown. First, 50 $\mu$l of the corresponding medium were added to the wells of the 96-well plate, the spaces between the wells were filled with 100 $\mu$l PBS each to prevent dehydration. Then a blank value was measured and the cells were added in corresponding numbers in 150 $\mu$l medium each, the final volume was 200 $\mu$l per well. The plate was placed in the "xCELLigence" and the impedance was measured at 37 °C and 5% $CO_2$ over 24 h every 30 min. The plate was then removed and a media change and treatment with the peptides were performed. The peptide concentrations corresponded to a 1:2 dilution series from 100 $\mu$M to 1.56 $\mu$M. A positive control was performed with 10 % tritone. Afterwards, the resistance at the bottom of the 96-well plate was measured every 30 min for a further 72 h. The resistance of the tritone was measured with a positive control of 10 % tritone. From the measured impedance values, the RTCA software calculated the so-called cell index according to the following formula:

$$ZI(t) = (R(f_n, t)- R(f_n, t_0))/Z_n$$

$ZI(t)$ = cell index at time t

$R(f_n,t)$ = measured impedance at frequency $f_n$ at time t

$R(f_n, t_0)$ = measured impedance at frequency $f_n$ at time $t_0$ (background measurement with medium without cells)

$Z_n$ = Corresponding frequency factor to frequency $f_n$

To determine the normalized cell index, the cell index at time t was divided by the value of the measurement after 24 hours. The measurement after 24 h was the last before the treatment with the peptides, so the comparability of the values was ensured independent of the total cell count.

**Experiments with Zebrafish**

To test HD5 (1-9) (SEQ ID No. 1) for toxicity and to assess its effect on embryonic development in zebrafish, embryos obtained from natural crosses of wildtype TE fish were used. After mating, embryos were incubated at 28 °C until 6 h postfertilization (hpf). Triplicates of five embryos each were then placed in wells of a 96-well plate containing 200 $\mu$l of different peptide concentrations. HD5 (1-9) (SEQ ID No. 1) dissolved in PBS was diluted in normal embryo medium (250 mg/L Instant Ocean salt, 1 mg/L methylene blue in reverse osmosis water adjusted to pH 7 with $NaHCO_3$) (Muller et al., "Differential diffusivity of Nodal and Lefty underlies a reaction-diffusion patterning system"; Science 336 (6082):721-724, 2012). The concentrations tested were 25 $\mu$M, 75 $\mu$M, 125 $\mu$M and 250 $\mu$M, and the concentration of PBS solvent was adjusted for each control group. At peptide concentrations of 75 $\mu$M and above we observed granulae in some wells, which could represent peptide accumulations. As a control, embryos were additionally incubated in 25 $\mu$g/ml cycloheximide (C4859, Sigma-Aldrich). After 13 hpf, 24 hpf and 48 hpf a microscopic phenotype analysis was performed. For each time point embryos were automatically imaged using an ACQUIFER Imaging Machine. For the phenotype analysis at 48 hpf, the larvae were manually dechorionated and anaesthetized with 2% tricaine methane-sulfonate (A5040-25G, Sigma-Aldrich). Images were acquired on an Axio Zoom.V16 microscope (ZEISS).

**Results**

In fig. 1, the results of MTT-assays showing that defensin-derived peptides have antiviral activity against HCMV and don't show toxicity in an MTT assay are shown. 10 000 HFF were seeded into each well of a 96-well-plate containing 200 $\mu$l of medium. Cells were incubated overnight at 37° C and 5 % CO2. Next day medium was changed to medium without Penicillin/Streptomycin (P/S). Cells were infected with TB40E-$\Delta$UL16-EGFP (MOI 0,5) and treated with peptides in concentrations of 7,5 $\mu$M and 75 $\mu$M in a volume of 100 $\mu$l. After 40 h of incubation cells were fixed with 2 % PFA in PBS, permeabilised with 90 % methanol in $H_2O$ and stained with DAPI. Infection rate was analysed (GFP+/DAPI+, normalized to solvent control, n=1).

Further, 10 000 HFF were seeded into each well of a 96-well-plate containing 200 $\mu$l of medium. Cells were incubated overnight at 37°C and 5 % $CO_2$. Next day medium was changed to medium without P/S and cells were treated with peptides in concentrations of 7,5 $\mu$M and 75 $\mu$M in a volume of 100 $\mu$l. After 40 h of incubation medium was changed and 10 $\mu$l of MTT stock solution was added. 3 h later medium and stock solution were removed and crystals were dissolved in 100 $\mu$l of 0,04 M hydrochloric acid in isopropyl. Absorption was measured (normalized to solvent control, n=3), and the results are shown in fig. 1B: (ns: not significant, **** $p < 0.0001$, *** $p < 0.001$, ** $p < 0.01$, * $p < 0.05$, upper symbol: 75 $\mu$M, lower symbol: 7,5 $\mu$M)).

As can be seen (see, fig. 1A), HD5 (1-9), a peptide according to the invention has an excellent antiviral effect in a concentration of 75 $\mu$M as compared to other tested peptides. Also, in un-infected corresponding cells (see fig. 1B); there was no negative effect of this peptide as compared to other peptides, even in a concentration of 75 $\mu$M.

In fig. 2, the results of experiments showing that HD5 (1-9) (= SEQ ID No. 1) inhibits HCMV infection of different cell types from a concentration of 50 $\mu$M and shows no cytotoxicity up to 150 $\mu$M are shown.

Different cell types were seeded into a 96-well-plate, each well containing 200 $\mu$l of medium (HFF and ARPE-19: 10000 cells per well, THP-1: 50 000 cells per well) (THP-1 is a monocyte cell type); all cell types are obtainable, e.g. at the ATCC (American Type Culture Collection). Cells were incubated overnight at 37° C and 5 % CO2. Next day medium was changed to medium without P/S. Cells were infected with TB40E-$\Delta$UL16-EGFP (HFF and ARPE-19: MOI 0,5; THP-1: MOI 10) and treated with peptide in concentrations from 1,56 $\mu$M to 100 $\mu$M in a volume of 100 $\mu$l. After 40 h of incubation cells were fixed with 2 % PFA in PBS and permeabilised with 90 % methanol in $H_2O$. Afterwards an antibody staining for IE-antigens was performed and nuclei were stained with DAPI. Infection rate was analysed and the results are shown in fig. 2A (IE+/DAPI+, normalized to medium control, HFF and ARPE-19: n=3, THP-1: n=1). As can be taken from fig. 2A, HD5 (1-9) displayed a HCMV-infection inhibitory effect on different infected cell types from a concentration of 50 $\mu$m.

Fig. 2B to D show the results for HFF (B), ARPE (C) and macrophages (D), which were seeded in a culture dish with a microelectrode network on the bottom (HFF and ARPE-19: 10 000 cells per well, macrophages: 20 000 cells per well, volume of 200 $\mu$l). Cells were incubated 24 h at 37°C and 5 % CO2. Next day medium was changed to medium without P/S and cells were treated with peptide in concentrations from 1,56 $\mu$M to 100 $\mu$M in a volume of 100 $\mu$l. Cells with peptide were incubated 72 h at 37°C and 5 % CO2. Impedance was measured via xCELLigence every 30 min, cell index was calculated from measured impedance and normalized to cell index after 24 h (HFF and ARPE-19: n=3, macrophages: n=1). As can be taken from fig. 2, a peptide of the invention, i.e. HD5 (1-9) does not show any cytotoxicity up to 150 $\mu$M on the tested different cell types.

Next, a peptide of the invention, i.e. HD5 (1-9) was tested on clinical HCMV isolates. 10 000 HFF were seeded into each well of a 96-well-plate containing 200 $\mu$l of medium. Cells were incubated overnight at 37° C and 5 % $CO_2$. Next day medium was changed to medium without P/S. Cells were infected with different clinical HCMV isolates (MOI 0,2) and treated with peptide in concentrations from 1,56 $\mu$M to 100 $\mu$M in a volume of 100 $\mu$l. After 40 h of incubation cells were fixed with 2% PFA in PBS and permeabilised with 90% methanol in $H_2O$. Afterwards an antibody staining for IE-antigens was performed and nuclei were stained with DAPI. Infection rate was analysed (IE+/DAPI+, normalized to medium control, n=1).

HD5(1-9) inhibits multiresistant, primary HCMV isolates

HCMV TB40/E is a lab-adapted strain that might differ from primary HCMV isolates in terms of cellular tropism, infectivity or cytopathic properties. We hence tested whether HD5 (1-9) (SEQ ID No. 1) is also active against primary HCMV isolates from different compartments of patients including amniotic fluid, breast milk and leukocytes (Fig. 3). Of note, the leukocyte isolate from a stem cell transplant recipient is genotypically (mutations UL97 L595S, UL54 V715M) and phenotypically resistant against GCV (IC50 > 30 $\mu$M), PFA (IC50 = 795 $\mu$M), and CDV (IC50 = 1.8 $\mu$M) (Gohring et al., 2013), while both isolates from amniotic fluid and breast milk were therapy-naive. We infected HFF cells at MOIs of 0.2 (Fig. 3A) and 0.1 (Fig. 3B) in the presence of increasing amounts of HD5 (1-9) (SEQ ID No. 1). Similar to our previous results, HD5 (1-9) (SEQ ID No. 1) inhibited HCMV infection efficiently at a concentration of 100 $\mu$M in HFF. Importantly, not only infection with the lab-adapted TB40/E strain was blocked, but also infection with the primary isolates was sensitive towards inhibition by HD5(1-9) (SEQ ID No. 1). Hence, HD5 (1-9) (SEQ ID No. 1) is a peptide inhibitor active against primary as well as multiresistant HCMV strains.

As can be taken from fig. 3, HD5 (1-9) also inhibited infection with all clinical isolates tested starting at a concentration of 50 $\mu$M.

Next, amino acid substitutions of a peptide according to the invention (HD5 (-9) were tested in view of alterations in their antiviral effects. In fig. 4, the results of experiments testing the antiviral activity of HD5 (1-9) and its derivatives comprising different amino acid substitutions on TB40E-$\Delta$UL16-EGFP infected HFF cells are shown.

10 000 HFF were seeded into each well of a 96-well-plate containing 200 $\mu$l of medium. Cells were incubated overnight at 37° C and 5 % $CO_2$. Next day medium was changed to medium without P/S and cells were infected with TB40E-$\Delta$UL16-EGFP (MOI 0,2). Cells were treated with HD5 (1-9) and its derivatives in concentrations from 1,56 $\mu$M to 100 $\mu$M (A) and HD5 (1-13) and its derivative (B) in concentrations of 7,5 $\mu$M and 75 $\mu$M in a volume of 100 $\mu$l. After 40 h of incubation cells were fixed with 2% PFA in PBS and permeabilised with 90% methanol in $H_2O$. Afterwards an antibody staining for IE-antigens was performed and nuclei were stained with DAPI. Infection rate was analysed (IE+/DAPI+, normalized to medium control, n=3). HD5 (1-9): ATCYCRTGR, (SEQ ID NO. 1) HD5 (1-13): ATCYCRTGRCATR (SEQ ID No. 3) ns: not significant, **** p < 0.0001, *** p < 0.001.

As can be taken from fig. 4A, the Arginine and Cysteine residues in a peptide according to the invention are crucial for the antiviral effect of HD5 (1-9), since alternations in these amino acids lead to a diminished, if not abolished, antiviral activity.

Next, experiments for identifying the specific mechanism of the antiviral effect of the peptides of the invention were assessed. In fig. 5, a diagram displaying results showing that HD5 (1-9) has an antiviral effect against HCMV infection by directly interacting with the cells is shown.

10 000 HFF were seeded into each well of a 96-well-plate containing 200 $\mu$l of medium. Cells were incubated overnight at 37°C and 5% $CO_2$. Next day medium was changed to medium without P/S. Cells were infected with TB40E-$\Delta$UL16-EGFP (MOI 0,2) and treated with peptide in concentrations from 1,56 $\mu$M to 100 $\mu$M in two different conditions: in the first condition there was no preincubation of virus and peptide, in the second condition the peptide in the corresponding concentrations was preincubated with the virus. In an additional condition the peptide was preincubated with the virus in 100 $\mu$M and then diluted to 10 $\mu$M in a volume of 100 $\mu$l. Preincubation was 1 h at 37°C. After 40 h of incubation cells were fixed with 2 % PFA in PBS and permeabilised with 90% methanol in $H_2O$. Afterwards an antibody staining for IE-antigens was performed and nuclei were stained with DAPI. Infection rate was analysed (IE+/DAPI+, normalized to medium control, n=3).

Also, in another experiment, 10 000 HFF were seeded into each well of a 96-well-plate containing 200 $\mu$l of medium. Cells were incubated overnight at 37°C and 5% $CO_2$. Next day medium was changed to medium without P/S. Cells were infected with TB40E-$\Delta$UL16-EGFP (MOI 0,2) and treated with peptide in concentrations from 1,56 $\mu$M to 100 $\mu$M in two different conditions: In the first condition HFF were preincubated with peptide in a volume of 60 $\mu$l 3 h at 37°C, peptide was concentrated in such a way, that after infection in a total volume of 100 $\mu$l concentrations from 1,56 $\mu$M to 100 $\mu$M were reached. In the second condition the peptide was added at the time of infection and in the third condition the peptide was added 3 h after infection. After 40 h of incubation cells were fixed with 2% PFA in PBS and permeabilised with 90% methanol in $H_2O$. Afterwards an antibody staining for IE-antigens was performed and nuclei were stained with DAPI. Infection rate was analysed (IE+/DAPI+, normalized to medium control, n=3).

With the results displayed in fig. 5, a direct interaction of the peptide of the invention with the cells was shown, rather than an inhibitory effect on the virus as it is the case with the presently available antiviral substances used for treating HCMV-infections.

In additional experiments, UL328 (encoding for pp150) was GFP (green fluorescence protein)-tagged, UL100 (encoding for gM) was mCherry-tagged. This allowed discrimination between enveloped particles, which were EGFP and mCherrypositive and appeared yellow, and non-enveloped particles, which were only EGFP-positive and appeared green.

HFF were preincubated with peptide in the corresponding concentration 1 h at 37°C and then infected TB40-$BAC_{KL7}$-UL32EGFP-UL 100mCherry. After 6 h of incubation, cells were fixed and stained with DAPI.

The results achieved with these experiments (data not shown) showed that HD5 (1-9) blocks the attachment of viral particles.

## Effect of HD5(1-9) on embryonic development

As a first test of HD5 (1-9) (SEQ ID No. 1) toxicity in vivo, as well as to elucidate potential effects of the peptide on embryonic development, experiments with zebrafish embryos were performed (He et al., 2014). Embryos were treated with HD5 (1-9) (SEQ ID No. 1) at concentrations of 25 $\mu$M, 75 $\mu$M, 125 $\mu$M and 250 $\mu$M starting at 6-7 h post fertilization (hpf), which is the time point recommended by pharmaceutical company-utilized assays to assess toxic effects of compounds on early embryonic development (Ball et al., 2014). Phenotypes were analyzed at 13, 24 and 48 hpf (data not shown). At the highest peptide concentration of 250 $\mu$M, half of the embryos had died by 13 hpf, indicating that excess of HD5 (1-9) (SEQ ID No. 1) can affect embryonic development. Impaired development at 250 $\mu$M peptide exposure was also evident over the whole observation period, leading to the death of 11 embryos and 4 embryos having severe developmental delays by 48 hpf. Reduction of the peptide concentration to 125 $\mu$M reduced the negative effects on embryonic development, with approximately half of the embryos having no alterations (data not shown). Furthermore, all embryos treated with 25 $\mu$M or 75 $\mu$M of HD5 (1-9) (SEQ ID No. 1) developed normally, except for one embryo that had died by 13 hpf due to unknown reasons. Altogether, HD5 (1-9) (SEQ ID No. 1) concentrations of 25 $\mu$M)-75 $\mu$M, which are higher than the IC50 of ~40 $\mu$M, do not affect zebrafish embryonic development or show toxicity *in vivo*.

**Claims**

1. A peptide for use in treating, inhibiting and/or preventing viral infections in mammals, wherein the viral infection is caused by Cytomegalovirus, wherein the peptide comprises a sequence having the following formula I:

$$A_1\text{-}B_1\text{-}X_1\text{-}C_1\text{-}X_2\text{-}Z_1\text{-}B_2\text{-}A_2\text{-}Z_2 \qquad \text{(Formula I)}$$

   wherein

   and $A_2$ are identical or different, and each $A_1$ and $A_2$ is a nonpolar amino acid
   having an aliphatic or basic side chain, and each is preferably Alanine or Glycine, $B_1$ and $B_2$ are identical or different, and each $B_1$ and $B_2$ is a polar amino acid and
   having a hydroxylated side chain, and is preferably selected from Serine, Threonine or Tyrosine,
   $C_1$ is an polar amino acid having a aromatic side chain, and is preferably Tyrosine,
   $X_1$ and $X_2$ each is Cysteine, and
   $Z_1$ and $Z_2$ each is Arginine.

2. A peptide for use in treating, inhibiting and/or preventing viral infections in mammals, wherein the viral infection is caused by a member of the family *Herpesviridae,* wherein the peptide has an amino acid sequence that consists of 9 successive amino acids having the sequence ATCYCRTGR (SEQ ID No. 1) or the reverse sequence of SEQ ID No. 1, RGTRCYCTA (SEQ ID No. 2).

3. The peptide for use of claim 1 or 2, wherein the peptide consists of the SEQ ID No. 1.

4. The peptide for use as claimed in any of claims 1 to 3, wherein the peptide is a chemically synthesized or a biologically expressed peptide.

5. The peptide for use as claimed in any of claims 2 to 4, wherein the member of the *Herpesviridae* family is a member of the *Betaherpesviridae* subfamily, and preferably is selected from at least one of the following: human Cytomegalovirus (HCMV; HHV-5), Herpes Simplex Virus 1 (HSV-1; HHV-1), Herpes Simplex Virus 2 (HSV-2; HHV-2), Varicella Zoster Virus (VZV; HHV-3), Epstein-Barr Virus (EBV; HHV- 4), Human Herpes Virus 6 (HHV-6), Human Herpes Virus 7 (HHV-7), or Kaposi's sarcoma-Associated Herpesvirus (KSHV; HHV-8).

6. The peptide for use as claimed in claim 1, wherein the Cytomegalovirus is human Cytomegalovirus (HCMV).

7. The peptide for use as claimed in claim 6, wherein the HCMV is a multiresistant HCMV strain.

8. The peptide for use as claimed in any of claims 1 to 7, wherein the peptide is used in combination with another anti-infective agent.

9. The peptide for use as claimed in any of claims 1 to 8, wherein the peptide is used in a unit dosage with an amount between 1 mg and 1000 mg.

10. A pharmaceutical composition for use in treating, preventing and/or inhibiting a *Herpesviridae* infection, the Pharmaceutical composition comprising a peptide as claimed in any of claims 2 or 3, and a pharmaceutically acceptable carrier, vehicle or diluent.

11. A pharmaceutical composition for use in treating, preventing and/or inhibiting a Cytomegalovirus infection, the pharmaceutical composition comprising a peptide as claimed in claim 1, and a pharmaceutically acceptable carrier, vehicle or diluent.

12. The pharmaceutical composition for use according to any one of claims 10 or 11, wherein the peptide is as defined in any one of claims 4, 8 or 9.

13. The peptide for use as claimed in any of claims 1 to 9 or the pharmaceutical composition for use as claimed in any one of claims 10 or 11, wherein the peptide or the pharmaceutical composition is administered to a subject that it selected from an immuno-compromised subject, pregnant women, newborns, or infants.

14. The peptide for use as claimed in claim 13, or the pharmaceutical composition for use of claim 13, wherein the immune-compromised subject is selected from the group consisting of retinitis patients, AIDS patients, transplant patients, solid organ transplantation recipients and allogeneic stem cell transplantation recipients; and the newborn is a CMV-infected neonate.

15. The pharmaceutical composition for use according to claim 10, wherein the pharmaceutical composition is used for treating, preventing or inhibiting an infection caused by a member of the *Betaherpesviridae,* and is preferably selected from at least one of the following: Cytomegalovirus (HCMV; HHV-5), Herpes Simplex Virus 1 (HSV-1; HHV-1), Herpes Simplex Virus 2 (HSV-2; HHV-2), Varicella Zoster Virus (VZV; HHV-3), Epstein-Barr Virus (EBV; HHV- 4), Human Herpes Virus 6 (HHV-6), Human Herpes Virus 7 (HHV-7), or Kaposi' sarcoma-Associated Herpesvirus (KSHV; HHV-8).

**Patentansprüche**

1. Peptid zur Verwendung beim Behandeln, Inhibieren und/oder Vorbeugen von Virusinfektionen bei Säugern, wobei die Virusinfektion durch das Cytomegalovirus hervorgerufen wird, wobei das Peptid eine Sequenz mit der folgenden Formel I umfasst:

$$A_1\text{-}B_1\text{-}X_1\text{-}C_1\text{-}X_2\text{-}Z_1\text{-}B_2\text{-}A_2\text{-}Z_2 \qquad \text{(Formel I)}$$

wobei

$A_1$ und $A_2$ gleich oder verschieden sind und $A_1$ und $A_2$ jeweils eine unpolare Aminosäure mit einer aliphatischen oder basischen Seitenkette sind und jeweils vorzugsweise Alanin oder Glycin sind,
$B_1$ und $B_2$ gleich oder verschieden sind und $B_1$ und $B_2$ jeweils eine polare Aminosäure mit einer hydroxylierten Seitenkette sind und vorzugsweise aus Serin, Threonin oder Tyrosin ausgewählt sind, $C_1$ eine polare Aminosäure mit einer aromatischen Seitenkette ist und vorzugsweise Tyrosin ist,
$X_1$ und $X_2$ jeweils Cystein sind und
$Z_1$ und $Z_2$ jeweils Arginin sind.

2. Peptid zur Verwendung beim Behandeln, Inhibieren und/oder Vorbeugen von Virusinfektionen bei Säugern, wobei die Virusinfektion durch ein Mitglied der Familie *Herpesviridae* hervorgerufen wird, wobei das Peptid eine Aminosäuresequenz aufweist, die aus 9 aufeinanderfolgenden Aminosäuren mit der Sequenz ATCYCRTGR (SEQ ID NO: 1) oder der umgekehrten Sequenz von SEQ ID NO: 1, RGTRCYCTA (SEQ ID NO: 2) besteht.

3. Peptid zur Verwendung nach Anspruch 1 oder 2, wobei das Peptid aus der SEQ ID NO: 1 besteht.

4. Peptid zur Verwendung nach einem der Ansprüche 1 bis 3, wobei das Peptid ein chemisch synthetisiertes oder ein biologisch exprimiertes Peptid ist.

5. Peptid zur Verwendung nach einem der Ansprüche 2 bis 4, wobei das Mitglied der *Herpesviridae*-Familie ein Mitglied der *Betaherpesviridae*-Unterfamilie ist und vorzugsweise aus mindestens einem von Folgendem ausgewählt ist: humanem Cytomegalovirus (HCMV; HHV-5), Herpes-simplex-Virus 1 (HSV-1; HHV-1), Herpes-simplex-Virus 2 (HSV-2; HHV-2), Varizella-Zoster-Virus (VZV; HHV-3), Epstein-Barr-Virus (EBV; HHV-4), humanem Herpesvirus 6 (HHV-6), humanem Herpesvirus 7 (HHV-7) oder Kaposi-Sarkom-assoziiertem Herpesvirus (KSHV; HHV-8).

6. Peptid zur Verwendung nach Anspruch 1, wobei das Cytomegalovirus das humane Cytomegalovirus (HCMV) ist.

**7.** Peptid zur Verwendung nach Anspruch 6, wobei das HCMV ein multiresistenter HCMV-Stamm ist.

**8.** Peptid zur Verwendung nach einem der Ansprüche 1 bis 7, wobei das Peptid in Kombination mit einem anderen Antiinfektivum verwendet wird.

**9.** Peptid zur Verwendung nach einem der Ansprüche 1 bis 8, wobei das Peptid in einer Einheitsdosis mit einer Menge zwischen 1 mg und 1000 mg verwendet wird.

**10.** Pharmazeutische Zusammensetzung zur Verwendung beim Behandeln, Vorbeugen und/oder Inhibieren einer *Herpesviridae*-Infektion, wobei die pharmazeutische Zusammensetzung ein Peptid nach einem der Ansprüche 2 oder 3 und einen pharmazeutisch verträglichen Träger, ein pharmazeutisch verträgliches Vehikel oder ein pharmazeutisch verträgliches Verdünnungsmittel umfasst.

**11.** Pharmazeutische Zusammensetzung zur Verwendung beim Behandeln, Vorbeugen und/oder Inhibieren einer Cytomegalovirus-Infektion, wobei die pharmazeutische Zusammensetzung ein Peptid nach Anspruch 1 und einen pharmazeutisch verträglichen Träger, ein pharmazeutisch verträgliches Vehikel oder ein pharmazeutisch verträgliches Verdünnungsmittel umfasst.

**12.** Pharmazeutische Zusammensetzung zur Verwendung nach einem der Ansprüche 10 oder 11, wobei das Peptid wie in einem der Ansprüche 4, 8 oder 9 definiert ist.

**13.** Peptid zur Verwendung nach einem der Ansprüche 1 bis 9 oder pharmazeutische Zusammensetzung zur Verwendung nach einem der Ansprüche 10 oder 11, wobei das Peptid oder die pharmazeutische Zusammensetzung einem Subjekt verabreicht wird, das aus einem immungeschwächten Subjekt, schwangeren Frauen, Neugeborenen oder Säuglingen ausgewählt ist.

**14.** Peptid zur Verwendung nach Anspruch 13 oder pharmazeutische Zusammensetzung zur Verwendung nach Anspruch 13, wobei das immungeschwächte Subjekt aus der Gruppe bestehend aus Retinitis-Patienten, AIDS-Patienten, Transplantationspatienten, Empfängern solider Organtransplantationen und Empfängern von Transplantationen allogener Stammzellen ausgewählt ist; und das Neugeborene ein CMV-infiziertes Neugeborenes ist.

**15.** Pharmazeutische Zusammensetzung zur Verwendung nach Anspruch 10, wobei die pharmazeutische Zusammensetzung zum Behandeln, Vorbeugen oder Inhibieren einer durch ein Mitglied der *Betaherpesviridae* hervorgerufenen Infektion verwendet wird und vorzugsweise aus mindestens einem von Folgendem ausgewählt ist: Cytomegalovirus (HCMV; HHV-5), Herpes-simplex-Virus 1 (HSV-1; HHV-1), Herpes-simplex-Virus 2 (HSV-2; HHV-2), Varizella-Zoster-Virus (VZV; HHV-3), Epstein-Barr-Virus (EBV; HHV-4), humanem Herpesvirus 6 (HHV-6), humanem Herpesvirus 7 (HHV-7) oder Kaposi-Sarkom-assoziiertem Herpesvirus (KSHV; HHV-8).

**Revendications**

**1.** Peptide destiné à être utilisé dans le traitement, l'inhibition et/ou la prévention des infections virales chez les mammifères, dans lequel l'infection virale est provoquée par le cytomégalovirus, dans lequel le peptide comprend une séquence répondant à la formule I suivante :

$$A_1\text{-}B_1\text{-}X_1\text{-}C_1\text{-}X_2\text{-}Z_1\text{-}B_2\text{-}A_2\text{-}Z_2 \qquad \text{(Formule I)}$$

dans lequel

$A_1$ et $A_2$ sont identiques ou différents, et chacun de $A_1$ et $A_2$ est un acide aminé non polaire ayant une chaîne latérale aliphatique ou basique, et chacun est de préférence alanine ou glycine,
$B_1$ et $B_2$ sont identiques ou différents, et chacun de $B_1$ et $B_2$ est un acide aminé polaire et possédant une chaîne latérale hydroxylée, et est de préférence choisi parmi la sérine, la thréonine ou la tyrosine,
$C_1$ est un acide aminé polaire ayant une chaîne latérale aromatique, et est de préférence la tyrosine,
$X_1$ et $X_2$ représentent chacun de la cystéine, et
$Z_1$ et $Z_2$ représentent chacun de l'arginine.

**2.** Peptide destiné à être utilisé dans le traitement, l'inhibition et/ou la prévention des infections virales chez les mammifères, dans lequel l'infection virale est provoquée par un membre de la famille des *Herpesviridae,* dans lequel le peptide a une séquence d'acides aminés qui consiste en 9 acides aminés successifs ayant la séquence ATCYCRTGR (SEQ ID No.1) ou la séquence inverse de SEQ ID No.1, RGTRCYCTA (SEQ ID No.2).

3. Peptide destiné à être utilisé selon la revendication 1 ou 2, dans lequel le peptide consiste en SEQ ID No.1.

4. Peptide destiné à être utilisé selon l'une quelconque des revendications 1 à 3, dans lequel le peptide est un peptide synthétisé chimiquement ou exprimé biologiquement.

5. Peptide destiné à être utilisé selon l'une quelconque des revendications 2 à 4, dans lequel le membre de la famille des *Herpesviridae* est un membre de la sous-famille des *Betaherpesviridae,* et est de préférence choisi parmi au moins l'un : du cytomégalovirus humain (HCMV ; HHV-5), du virus de l'herpès simplex 1 (HSV-1 ; HHV-1), du virus de l'herpès simplex 2 (HSV-2 ; HHV-2), du virus varicelle-zona (VZV ; HHV-3), du virus d'Epstein-Barr (EBV ; HHV-4), du virus de l'herpès humain 6 (HHV-6), du virus de l'herpès humain 7 (HHV-7) ou du virus de l'herpès associé au sarcome de Kaposi (KSHV ; HHV-8).

6. Peptide destiné à être utilisé selon la revendication 1, dans lequel le cytomégalovirus est le cytomégalovirus humain (HCMV).

7. Peptide destiné à être utilisé selon la revendication 6, dans lequel le HCMV est une souche de HCMV multirésistante.

8. Peptide destiné à être utilisé selon l'une quelconque des revendications 1 à 7, dans lequel le peptide est utilisé en combinaison avec un autre agent anti-infectieux.

9. Peptide destiné à être utilisé selon l'une quelconque des revendications 1 à 8, dans lequel le peptide est utilisé en dose unitaire avec une quantité comprise entre 1 mg et 1 000 mg.

10. Composition pharmaceutique destinée à être utilisée dans le traitement, la prévention et/ou l'inhibition d'une infection à *Herpesviridae,* la composition pharmaceutique comprenant un peptide selon l'une quelconque des revendications 2 ou 3, et un support, véhicule ou diluant pharmaceutiquement acceptable.

11. Composition pharmaceutique destinée à être utilisée dans le traitement, la prévention et/ou l'inhibition d'une infection à cytomégalovirus, la composition pharmaceutique comprenant un peptide selon la revendication 1, et un support, véhicule ou diluant pharmaceutiquement acceptable.

12. Composition pharmaceutique destinée à être utilisée selon l'une quelconque des revendications 10 ou 11, dans laquelle le peptide est tel que défini dans l'une quelconque des revendications 4, 8 ou 9.

13. Peptide destiné à être utilisé selon l'une quelconque des revendications 1 à 9 ou composition pharmaceutique destinée à être utilisée selon l'une quelconque des revendications 10 ou 11, dans lequel le peptide ou la composition pharmaceutique est administré à un sujet sélectionné parmi un sujet immunodéprimé, les femmes enceintes, les nouveau-nés ou les nourrissons.

14. Peptide destiné à être utilisé selon la revendication 13, ou composition pharmaceutique destinée à être utilisée selon la revendication 13, dans lequel le sujet immunodéprimé est choisi dans le groupe constitué de patients atteints de rétinite, de patients atteints du SIDA, de patients transplantés, de receveurs de transplantation d'organes solides et de receveurs de transplantation de cellules souches allogéniques ; et le nouveau-né est un nouveau-né infecté par le CMV.

15. Composition pharmaceutique destinée à être utilisée selon la revendication 10, dans laquelle la composition pharmaceutique est utilisée dans le traitement, la prévention ou l'inhibition d'une infection provoquée par un membre des *Betaherpesviridae,* et est de préférence choisie parmi au moins l'un : du cytomégalovirus (HCMV ; HHV-5), du virus de l'herpès simplex 1 (HSV-1 ; HHV-1), du virus de l'herpès simplex 2 (HSV-2 ; HHV-2), du virus varicelle-zona (VZV ; HHV-3), du virus d'Epstein-Barr (EBV ; HHV-4), du virus de l'herpès humain 6 (HHV-6), du virus de l'herpès humain 7 (HHV-7) ou du virus de l'herpès associé au sarcome de Kaposi (KSHV ; HHV-8).

Fig. 1

Fig. 2

Fig. 3

Fig. 4

Fig. 5

**EP 3 965 793 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **STAWIKOWSKI et al.** Introduction to peptide synthesis. *Cur. Prot. Prot. Sci.,* 2012, (69), 18.1.1-18.1.13
- **WEGMULLER ; SCHMID.** Recombinant Peptide Production in Microbial Cells. *Current Organic Chemistry,* 2014, vol. 18 (8
- **DAVIS et al.** *Basic Methods in Molecular Biology,* 1986
- Handbook of Pharmaceutical Excipients. Pharmaceutical Press, 2012
- **TISCHER et al.** Two-step red-mediated recombination for versatile high-efficiency markerless DNA manipulation in Escherichia coli. *Biotechniques,* 2006, vol. 40 (2), 191-7
- **SINZGER et al.** Macrophage cultures are susceptible to lytic productive infection by endothelial-cell-propagated human cytomegalovirus strains and present viral IE1 protein to CD4+ T cells despite late downregulation of MHC class II molecules. *J. Gen. Virol.,* 2006, vol. 87, 1853-62

- **GOHRING et al.** Dynamics of coexisting HCMV-UL97 and UL54 drug-resistance associated mutations in patients after haematopoietic cell transplantation. *J. Clin. Virol.,* 2013, vol. 57 (1), 43-49
- **BERRIDGE et al.** Characterization of the cellular reduction of 3-(4,5-dimethylthiazol-2-yl)-2,5-diphenyltetrazolium bromide (MTT): subcellular localization, substrate dependence, and involvement of mitochondrial electron transport in MTT reduction. *Arch. Biochem. Biophys.,* 1993, vol. 303 (2), 474-82
- **DIEMERT et al.** Impedance measurement for real time detection of neuronal cell death. *J Neurosci Methods,* 2012, vol. 203 (1), 69-77
- **MULLER et al.** Differential diffusivity of Nodal and Lefty underlies a reaction-diffusion patterning system. *Science,* 2012, vol. 336 (6082), 721-724